# EUROPÄISCHE PATENTANMELDUNG

(11) **EP 1 184 453 A1**
(43) Veröffentlichungstag der Anmeldung: **06.03.2002**
(21) Anmeldenummer: 00118808.5
(22) Anmeldetag: 31.08.2000
(51) Int. Cl.: C12F 3/06, A23L 1/212, A61K 7/48

(54) **Mittel zur Nahrungsergänzung und zur Körperbehandlung**

(71) Anmelder: Krug, Stefanija, 54290 Trier (DE); Reinfurth, Volker, 54292 Trier (DE)
(72) Erfinder: Krug, Stefanija, 54290 Trier (DE); Reinfurth, Volker, 54292 Trier (DE)
(74) Vertreter: Dr. Weitzel & Partner

(57) **Zusammenfassung**

Die Erfindung betrifft ein Mittel zur Nahrungsergänzung und Körperbehandlung.

Die Erfindung ist dadurch gekennzeichnet, daß das Mittel durch von der Maische getrennte Weintraubenkerne gebildete oder aus Weintraubenkernen hergestellte Feststoffteile enthält.

## Beschreibung

Die Erfindung betrifft ein Mittel zur Nahrungsergänzung und Körperbehandlung sowie ein Verfahren zur Herstellung des Mittels.

Beim Weinkeltern anfallende Maische wird nur in geringem Umfang z.B. zur Herstellung von Trestern genutzt. Darüber hinaus ist es bekannt, aus den in der Maische enthaltenen Weintraubenkernen zur Ernährung von Menschen und Tier verwendbares Öl zu gewinnen.

Der Erfindung liegt die Aufgabe zugrunde, die beim Keltern anfallenden Maischrückstände über den oben aufgezeigten Umfang hinaus nutzbar zu machen.

Diese Aufgabe wird durch ein Mittel zur Nahrungsergänzung und Körperbehandlung gelöst, welches durch von der Maische getrennte Weintraubenkerne gebildete oder aus Weintraubenkernen hergestellte Feststoffteile enthält.

Die Erfindung sieht also die Einbeziehung von Weintraubenkernen als solche oder in verarbeiteter Form in die Ernährung von Mensch und Tier vor, wobei die in den Weintrauben bzw. dem Verarbeitungsprodukt enthaltenen Substanzen, unter anderem Spurenelemente, die Ernährung vorteilhaft ergänzen und eine gesundheits- und leistungsfördernde Wirkung ausüben können.

Seit einiger Zeit wird in verstärktem Maße ökologischer Weinbau betrieben. Dabei sind relativ strenge Regeln bezüglich der Düngung der Rebe und/oder der Behandlung beim Pressen und sonstigen Weiterverarbeiten einzuhalten. So sind gewisse Chemikalien nicht oder nur sehr bedingt zugelassen. Die Erfinder haben erkannt, daß die erfindungsgemäßen Mittel ihre vorteilhafte Wirkung in besonderem Maße entfalten, wenn bei deren Herstellung die Regeln der Ökologie eingehalten werden.

In einer bevorzugten Ausführungsform der Erfindung werden die Feststoffteile durch Pressen der Weintraubenkerne und anschließende Zerkleinerung des Preßgutes hergestellt. Durch das Pressen werden die Traubenkerne einerseits wenigstens teilweise entölt, wodurch sich die Haltbarkeit des durch die Verarbeitung der Weintraubenkerne erzeugten Produkts erhöht. Andererseits wird durch das Pressen unter wenigstens teilweiser Aufhebung der Kornstruktur im Preßgut eine die Weintraubenkerne umgebende Hülle zerstört, welche den Aufschluß der Traubenkerne bei der Nahrungsverwertung im menschlichen und tierischen Körper erschwert.

In der bevorzugten Ausführungsform der Erfindung sind die Feststoffteile Pulverartikel, d.h. die Weintraubenkerne oder das Preßgut werden zu einem feinen Mehl vermahlen, wobei die Zerkleinerung vorzugsweise in mehreren Stufen erfolgt.

Die Feststoffteile und insbesondere die Pulverartikel können, ggf. unter Zusatz eines Bindemittels, zu einer Tablette verpreßt werden. In einer anderen Darreichungsform sind die Feststoffteile in einer Kapsel oder Einschweißpackung enthalten.

Schließlich können die Feststoffteile in einer auf die menschliche Haut aufzutragenden Creme enthalten sein, z.B. eines Gesichts- oder Körperpeelings.

Die Erfindung ist anhand der Zeichnungen erläutert. Darin sind Schritte eines Verfahrens zur Pulverisierung von Weintraubenkernen dargestellt.

Man erkennt eine Trenneinrichtung 1, welcher beim Keltern anfallende Maische zugeführt wird und welche von der Maische die darin enthaltenen Traubenkerne trennt. Als solche Trenneinrichtungen können in handelsüblichen Schleudertrommeln enthaltene Separatoren verwendet werden.

Die Trennung muß innerhalb weniger Stunden nach dem Keltern erfolgen, um Schimmelbefall der Maische zu vermeiden. Die mit Hilfe der Trenneinrichtung 1 gewonnenen Kerne sind, ebenfalls zur Vermeidung von Schimmelbefall, möglichst schnell zu trocknen und nachfolgend ggf. einer Reinigung unterziehen, wie dies bei Getreide üblich ist. Unter Umständen kann auch eine Schädlinge abtötende Begasung erfolgen.

Die getrockneten und ggf. einer Reinigung unterzogenen Traubenkerne werden im nächsten Verarbeitungsschritt einer Schneckenpresse 2 zugeführt, wo eine Entölung erfolgt und die Hüllen der Traubenkerne zerstört werden. Der Ölanteil von Traubenkernen liegt bei rund 14 Gew.-%. In dem hier beschriebenen Ausführungsbeispiel wird mit Hilfe der Schneckenpresse 2 etwa die Hälfte des in den Traubenkernen enthaltenen Öls abgepreßt, d.h. der Ölanteil auf etwa 6 bis 7 % verringert. Von der Schneckenpresse 2 ausgepreßte, teilentölte Preßlinge 3 werden in einem nachgeordneten Häcksler 4 wieder zerkleinert. Anstelle eines Häcklers könnte zur Zerkleinerung der Preßlinge auch eine Hammermühle eingesetzt werden.

Die zerkleinerten Preßlinge werden dann in einem Walzenstuhl 5 zu einem braunen Pulver 6 vermahlen. Über nicht gezeigte, z.B. durch Plansichter oder Taumelsiebe gebildete Siebeinrichtungen läßt sich eine gewünschte Korngrößenverteilung erreichen.

Das Pulver 6 enthält eine Vielzahl für die Ergänzung der Ernährung von Mensch und Tier wertvolle Stoffe, insbesondere Spurenelemente. Die nachfolgende Tabelle enthält einen Analysenbefund.

| | | | | | |
|---|---|---|---|---|---|
| Saccharose | 0,9 | g/100 g | Glucose | 1,2 | g/100 g |
| Fructose | 2,1 | g/100 g | Calzium AAS | 540 | mg/100 g |
| Magnesium AAS | 105 | mg/100 g | Natrium AAS | 10,4 | mg/100 g |
| Kalium AAS | 500 | mg/100 g | 3,5 Dichloranilin | n, n, | µg/l |
| Pyrimethanil | n, n, | µg/l | Vinciozolin | n, n, | µg/l |
| Parathlon-Methyl | n, n, | µg/l | Dichlorfluanid | n, n, | µg/l |
| Diethofencarb | n, n, | µg/l | ParathioNahrung sergänzungthyl | n, n, | µg/l |
| Triadimefon | n, n, | µg/l | Penconazole | n, n, | µg/l |
| Tolyfluanid | n, n, | µg/l | Triademenol | n, n, | µg/l |
| Procymidone | n, n, | µg/l | Methidathion | n, n, | µg/l |
| Pyrifenox | n, n, | µg/l | Fludioxonil | n, n, | µg/l |
| Iprodione | n, n, | µg/l | Phosalone | n, n, | µg/l |
| Fenamirol | n, n, | µg/l | Fluqinconazol | n, n, | µg/l |
| Dimethomorph | n, n, | µg/l | Eisen (Fe) | 11,6 | mg/100 g |
| Kupfer (CU) | 1,0 | mg/100 g | Zink (Zn) | 3,2 | mg/100 g |
| Selen(Se) | 0,10 | mg/100 g | | | |

Das gewonnene Pulver kann z.B. zur Ergänzung des Tagesbedarfs an Spurenelementen Nahrungsmitteln zugesetzt und z.B. beim Kochen und Backen als Zusatz verwendet werden.

Das Traubenpulver läßt sich, ggf. unter Zusatz eines Bindemittels, wie z.B. Saccharose, zu Dragees verpressen, In einer anderen Darreichungsform ist das Traubenpulver in Kapseln oder einer Einschweißpackung enthalten.

Die in Traubenkernen enthaltenen Stoffe vermögen im menschlichen und tierischen Körper in vielfältiger Weise positiv zu wirken. Unter anderem fördern sie Konzentration, Potenz, Durchblutung, Immunabwehr und damit nicht zuletzt das allgemeine Wohlbefinden. Besonders geeignet ist Traubenpulver zur Ergänzung der Nahrung von Sportlern, um oxidativen Streß abzubauen und die Leistung zu steigern. Die konzentrationsfördernde Wirkung kann Schülern, vor allem solchen mit Lernschwierigkeiten, zugute kommen. Schließlich wirken in dem Traubenpulver enthaltene Antioxidanzien und Spurenelemente dem Haarausfall entgegen. Durch Förderung der Kollagenbildung kommt es zur Faltenrückbildung und damit zur Verzögerung des Alterungsprozesses.

Eine Besserungswirkung tritt ferner bei bestimmten Krankheitsbildern ein, wobei hier Hautkrankheiten, wie Akne und Schuppenflechte, Diabetes, Erkrankungen des Knochengewebes, Rheuma, Osteoporose sowie Herzkreislauf- und Gefäßerkrankungen zu nennen sind.

Es ist denkbar, das Traubenpulver als Instantpulver, z.B. als Zusatz von Tee und Erfrischungsgetränken zu verwenden.

Das Traubenpulver kann ferner in einer Creme verwendet werden, z.B. zur Herstellung eines Gesichts- und/oder Körperpeelings, zur Herstellung einer Körpercreme, die insbesondere zur Beruhigung der Haut nach dem Sonnenbad dient, oder einer Gesichtsmaskencreme, welche einer Faltenbildung vorbeugt und Faltenrückbildung fördert.

Positive Wirkungen sind auch bei Ergänzung der Haustiernahrung durch das Traubenkernprodukt zu erwarten, wobei hierfür statt feinem Mehl grob zerkleinerte Preßlinge aus gepreßten Traubenkernen verfüttert werden können.

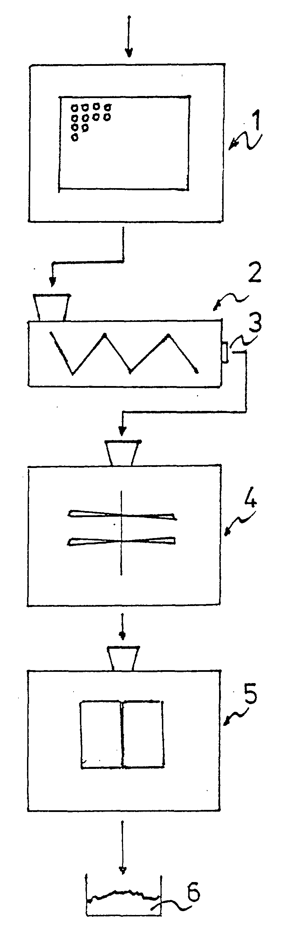

## Patentansprüche

1. Mittel zur Nahrungsergänzung und Körperbehandlung, **dadurch gekennzeichnet, daß** das Mittel durch von der Maische getrennte Weintraubenkerne gebildete oder aus Weintraubenkernen hergestellte Feststoffteile enthält.

2. Mittel nach Anspruch 1, **dadurch gekennzeichnet, daß** die Weintraubenkerne aus ökologischem Weinanbau gewonnen wurden.

3. Mittel nach Anspruch 1 oder 2, **dadurch gekennzeichnet, daß** die Feststoffteile durch Pressen (2) der Weintraubenkerne und Zerkleinern (4, 5) des Preßgutes (3) hergestellt sind.

4. Mittel nach einem der Ansprüche 1 bis 3, **dadurch gekennzeichnet, daß** die Feststoffteile, ggf. durch das Pressen, wenigstens teilweise entölt sind.

5. Mittel nach einem der Ansprüche 1 bis 4, **dadurch gekennzeichnet, daß** die Feststoffteile Pulverartikel sind.

6. Mittel nach einem der Ansprüche 1 bis 5, **dadurch gekennzeichnet, daß** die Feststoffteile, ggf. unter Zusatz eines Bindemittels, zu einer Tablette verpreßt sind.

7. Mittel nach einem der Ansprüche 1 bis 5, **dadurch gekennzeichnet, daß** die Feststoffteile in einer Kapsel oder Einschweißpackung enthalten sind.

8. Mittel nach einem der Ansprüche 1 bis 5, **dadurch gekennzeichnet, daß** die Feststoffteile in einer Creme enthalten sind.

9. Verfahren zur Herstellung eines Mittels zur Nahrungsergänzung und Körperbehandlung bei dem Weintraubenkerne nach dem Keltern von der Maische getrennt und gepreßt werden, **dadurch gekennzeichnet, daß** das Preßgut (3) ferner zerkleinert wird.

10. Verfahren nach Anspruch 9, **dadurch gekennzeichnet, daß** die Weintraubenkerne aus ökologischem Weinanbau gewonnen wurden.

11. Verfahren nach Anspruch 9 oder 10, **dadurch gekennzeichnet, daß** die Weintraubenkerne unter wenigstens teilweiser Entölung gepreßt werden.

12. Verfahren nach einem der Ansprüche 9 bis 11, **dadurch gekennzeichnet, daß** das Preßgut, vorzugsweise in mehreren Zerkleinerungsstufen, pulverisiert wird.

13. Aus Traubenkernen hergestelltes Produkt, gekennezichnet durch Feststoffteile, die durch Zerkleinern der Weintraubenkerne oder/und durch Pressen der Weintraubenkerne und Zerkleinerung des Preßgutes gebildet sind.

14. Produkt nach Anspruch 13, **dadurch gekennzeichnet, daß** die Weintraubenkerne aus ökologischem Weinanbau gewonnen wurden.

15. Produkt nach Anspruch 13 oder 14, **dadurch gekennzeichnet, daß** die Traubenkerne oder das Preßgut zu Traubenkernmehl zerkleinert sind.
